# EUROPÄISCHE PATENTSCHRIFT

(11) **EP 1 322 355 B1**
(45) Veröffentlichungstag und Bekanntmachung des Hinweises auf die Patenterteilung: **27.10.2004**
(21) Anmeldenummer: 01951308.4
(22) Anmeldetag: 31.07.2001
(51) Int. Cl.: A61M 5/315

(54) **VORRICHTUNG ZUR DOSIERTEN VERABREICHUNG EINES INJIZIERBAREN PRODUKTS**
DEVICE FOR CARRYING OUT THE DOSED ADMINISTRATION OF AN INJECTABLE PRODUCT
DISPOSITIF D'ADMINISTRATION DOSEE D'UN PRODUIT INJECTABLE

(30) Priorität: 19.09.2000 DE 10046279
(43) Veröffentlichungstag der Anmeldung: 02.07.2003
(73) Patentinhaber: TecPharma Licensing AG, 3401 Burgdorf (CH)
(72) Erfinder: HEINIGER, Hanspeter, CH-4932 Lotzwil (CH); GEISER, Simone, CH-4900 Langenthal (CH)
(86) Internationale Anmeldenummer: PCT/CH2001/000468
(87) Internationale Veröffentlichungsnummer: WO 2002/024260

(56) Entgegenhaltungen:
- US-A- 5 112 317
- US-A- 5 611 783

## Beschreibung

Die Erfindung betrifft eine Vorrichtung für eine dosierte Verabreichung eines injizierbaren Produkts mit einer Dosiereinrichtung, die nach Auswahl einer zu verabreichenden Produktdosis zurückgesetzt werden kann.

Bei bekannten Vorrichtungen, beispielsweise mit Spindeltrieben oder Zahnstangentrieben, ist eine Rücksetzung der Dosiereinrichtung für einen Verwender der Vorrichtung entweder sehr umständlich oder überhaupt nicht möglich. Eine Rücksetzung kann beispielsweise den Austausch eines entleerten Produktbehältnisses erleichtern, wie dies in der EP 0 614 386 B1 beschrieben wird. Hierzu ist eine Zahnstange, die auf einen in dem Behältnis angeordneten Kolben wirkt, nur über einen Teil ihres Umfangs mit Zähnen versehen. Die Zähne der Zahnstange wirken mit Gegenzähnen derart zusammen, dass ein Verschieben der Zahnstange zum Zwecke der Produktausschüttung in diskreten Schritten möglich ist, aber ein einfaches Zurückschieben der Zahnstange verhindert wird. Für ein Zurücksetzen der Zahnstange ist die Zahnstange so ausgebildet, dass die Zähne und die Gegenzähne durch Drehung der Zahnstange um 90° außer Eingriff und die Zahnstange anschließend manuell zurückgezogen und in eine Ausgangsstellung für eine erneute, dosierte Ausschüttung gebracht werden können. Das Zurücksetzen des Abtriebsglieds erfordert von einem Verwender die koordinierte Ausführung mehrerer Bewegungen. In der Verabreichung von medizinisch wirksamen Produkten, einem bevorzugten Anwendungsgebiet der Erfindung, kann dies für Verwender, die möglicherweise unter feinmotorischen Störungen leiden, recht schwierig werden.

Die US 5 112 317 umfasst die Merkmale der Präembel des Anspruchs 1.

Es ist eine Aufgabe der Erfindung, bei einer Vorrichtung zur dosierten Verabreichung eines injizierbaren Produkts eine Rücksetzung mit möglichst wenigen und möglichst einfachen Handgriffen zu ermöglichen.

Diese Aufgabe wird durch den Gegenstand von Anspruch 1 gelöst. Vorteilhafte Ausgestaltung und Weiterbildungen werden durch die abhängigen Ansprüche beschrieben.

Bei Vorrichtungen, wie die Erfindung sie betrifft, ist in einem Gehäuse ein Behältnis aufgenommen, welches das Produkt enthält und für eine Ausschüttung einer ausgewählten Produktdosis einen Kolben auf einen Behältnisauslass zu verschiebbar aufnimmt. Zum Auswählen der Produktdosis wirken ein Dosierglied und eine Antriebseinheit zusammen. Das Dosierglied führt für das Auswählen der Produktdosis eine Dosierbewegung relativ zu dem Gehäuse aus. Die Antriebseinheit ist mit dem Gehäuse und dem Dosierglied derart gekoppelt, dass durch die Dosierbewegung des Dosierglieds eine Verstellbewegung der Antriebseinheit aus einer Dosierausgangsstellung in eine Dosierendstellung relativ zu dem Gehäuse bewirkt wird. Die Vorrichtung weist ferner eine Betätigungseinrichtung auf, welche die Antriebseinheit veranlasst, eine Ausschüttbewegung auszuführen, durch die der Kolben auf den Behältnisauslass zu vorgeschoben und dadurch die ausgewählte Produktdosis ausgeschüttet und verabreicht wird.

Die Verstellbewegung der Antriebseinheit aus der Dosierausgangsstellung in die Dosierendstellung oder zumindest in Richtung auf die Dosierendstellung zu ist vorzugsweise eine von der Ausschüttbewegung separate, reine Verstellbewegung relativ zu dem Gehäuse und relativ zu dem Kolben. Durch die separate Verstellbewegung wird ein lichter Abstand zwischen dem Kolben und einem Abtriebsglied der Antriebseinheit in einer Dosierstellung der Vorrichtung verringert. Bei der Ausschüttbewegung drückt das Abtriebsglied gegen den Kolben. Anstatt mit der Verstellbewegung das Abtriebsglied relativ zu dem Kolben zu bewegen, kann die Verstellbewegung aber auch von einem anderen Glied der Antriebseinheit, das die Ausschüttbewegungen zusammen mit dem Abtriebsglied ausführt, relativ zu dem Abtriebsglied ausgeführt werden. Es kann auch das Dosierglied die Verstellbewegung ausführen, indem das Dosierglied bei seiner Dosierbewegung gleichzeitig eine Verstellbewegung relativ zu einem gehäuseseitigen, mechanischen Anschlag ausführt und dadurch ein Hub der Ausschüttbewegung definiert wird. Das Dosierglied wäre in dieser Ausführung auch Bestandteil der Antriebseinheit, indem es deren Ausschüttbewegung mitmacht. Durch die Trennung von Verstellbewegung und Ausschüttbewegung ist eine besonders sichere Dosisauswahl möglich. Ferner kann die Ausschüttbewegung stets die gleiche bleiben. Für diese bevorzugte Art der Dosierung haben sich Spindeltriebe besonders bewährt. Obgleich weniger bevorzugt, können die Verstellbewegung und die Ausschüttbewegung der Antriebseinheit auch zusammenfallen, wenn beispielsweise die zu verabreichende Produktdosis noch während der Ausschüttbewegung gewählt wird, wie dies beispielsweise bei einer als Zahnstangentrieb ausgebildeten Antriebseinheit möglich ist.

Erfindungsgemäß weist die Vorrichtung eine Rücksetzfeder auf, die in einem gespannten Zustand gesichert ist und durch Entsicherung mit der Antriebseinheit gekoppelt wird. Durch die Kopplung bewirkt die sich entladende Federenergie eine Rücksetzbewegung der Antriebseinheit in Richtung auf ihre Dosierausgangsstellung zu. Es muss lediglich die Sicherung der Rücksetzfeder gelöst werden, was mit einem einfachen Knopfdruck oder einem einfachen Verschieben eines Schiebers mit nur einer Hand erfolgen kann.

Die Vorrichtung kann bereits von einem Hersteller mit gespannter Rücksetzfeder bereitgestellt werden, so dass die Rücksetzfeder sich im Bedarfsfall nur noch entlädt. Vorzugsweise wird die Rücksetzfeder jedoch gespannt, wenn die Antriebseinheit auf ihre Dosierendstellung zu verstellt wird. In diesem Fall ist eine Blockiereinrichtung vorgesehen, die die Verstellbewegung zulässt, aber verhindert, dass die Antriebseinheit unter der Einwirkung der Rücksetzfeder versehentlich in Richtung auf ihre Dosierausgangsstellung zu zurückbewegt wird. Vorzugsweise wirkt die Rücksetzfeder über das Dosierglied auf die Antriebseinheit. Auch die Blockiereinrichtung wirkt vorzugsweise auf das Dosierglied.

Besonders bevorzugt bildet eine mechanische Feder, insbesondere eine Spiralfeder, die Rücksetzfeder. Wird zum Zwecke der Verstellung der Antriebseinheit ein Glied der Antriebseinheit relativ zu dem Gehäuse verdreht, so kann die Spiralfeder zwischen dem Gehäuse und diesem Glied der Antriebseinheit eingespannt sein. Bevorzugter ist solch eine Spiralfeder zwischen dem Gehäuse und dem Dosierglied eingespannt, das in diesem Falle zur Ausführung der Dosierbewegung relativ zu dem Gehäuse drehbar gelagert ist.

In bevorzugten Ausführungen handelt es sich bei der Antriebseinheit um einen Spindeltrieb. Eine Drehbewegung des Dosierglieds wird in den Spindeltrieb eingeleitet, und der Spindeltrieb führt die Verstellbewegung aus. Der Spindeltrieb kann insbesondere mehrstufig ausgeführt sein, wie dies in der WO 98/47552 beschrieben ist, deren Offenbarung hiermit in Bezug auf vorteilhafte Spindeltriebe und noch allgemeiner in Bezug auf vorteilhafte, mehrstufige Antriebseinheiten in Bezug genommen wird.

Die Rücksetzfeder kann mit Vorteil nur dazu verwendet werden, die Antriebseinheit bis in eine Dosierausgangsstellung zurück zu setzen, die es vor einer ersten Verstellung eingenommen hat. Falls ein Spindeltrieb verwendet wird, entfällt das oft als lästig empfundene, jedoch für eine Wiederverwendung unerlässliche Zurückdrehen der Antriebseinheit.

Alternativ kann mit der erfindungsgemäßen Rücksetzfeder auf sichere Weise eine versehentlich ausgewählte Überdosis korrigiert werden. In besonders bevorzugten Ausführungen wird die Rücksetzfeder sowohl für eine vollständige Rücksetzung als auch für eine teilweise Rücksetzung der Antriebseinheit verwendet

Die genannte Blockiereinrichtung weist ein Blockierglied und ein Blockiergegenglied auf, die in einem gegenseitigen Blockiereingriff eine Verstellung der Antriebseinheit in Richtung auf ihre Dosierausgangsstellung zu verhindern, aber eine Verstellung in Richtung auf ihre Dosierendstellung zu zulassen. Das Blockierglied ist mit der Antriebseinheit gekoppelt, vorzugsweise steif Vorzugsweise ist das Blockiergegenglied mit dem Gehäuse verdrehgesichert, aber auf das Blockierglied zu und von dem Blockierglied weg verschiebbar verbunden, wobei es von einer Federkraft auf das Blockierglied zu belastet wird. Der Blockiereingriff wird vorteilhafterweise durch eine Rastverbindung mit mehreren Raststellungen, welche das Blockierglied und das Blockiergegenglied relativ zueinander einnehmen können, gebildet. Dies erlaubt die Auswahl der auszuschüttenden Produktdosis in diskreten Schritten.

Um in bevorzugten Ausführungen eine versehentliche Überdosierung korrigieren zu können, ist ein Entsicherungselement vorgesehen, durch dessen Betätigung der Blockiereingriff gelöst werden kann. Um eine schrittweise Korrektur zu ermöglichen, d.h. um das Blockierglied und das Blockiergegenglied relativ zueinander aus der gerade eingenommenen, letzten Raststellung in die unmittelbar davor eingenommene, vorletzte Raststellung überführen zu können, bildet das Blockierglied einen mechanischen Anschlag aus, der mit dem Entsicherungselement zusammenwirkt. Nachdem der Blockiereingriff in der letzten Raststellung durch Betätigung des Entsicherungselements gelöst worden ist, wird das Blockierglied durch die Federkraft der Rücksetzfeder in Richtung auf die vorletzte Raststellung zu zurückbewegt. Der Anschlag des Blockierglieds ist so ausgebildet, dass der Anschlag gegen das Entsicherungselement stößt, bevor das Blockierglied und das Blockiergegenglied sich über ihre vorletzte Raststellung hinaus zurückbewegt haben. Das Entsicherungselement erfüllt gleichzeitig die Aufgabe des Lösens des Blockiereingriffs und der Ausbildung eines mechanischen Anschlags zur Begrenzung der Rückwärtsbewegung des Blockierglieds und damit letztlich der Rücksetzbewegung der Antriebseinheit. Indem das Blockierglied das Blockiergegenglied in einer Übertragungsstrecke zwischen der Rücksetzfeder und der Antriebseinheit angeordnet sind, kann die Rücksetzfeder nicht nur zum vollständigen Rücksetzen der Antriebseinheit, sondern auch zur schrittweisen Verringerung der Produktdosis verwendet werden.

In bevorzugten Ausführungen sind das Dosierglied, die Rücksetzfeder und die Blockiereinrichtung neben der Antriebseinheit angeordnet und führen die Ausschüttbewegung der Antriebseinheit vorzugsweise ebenfalls aus. Antriebseinheit, Dosierglied, Rücksetzfeder und Blockiereinrichtung werden vorzugsweise im Ganzen bewegt und verändern nicht ihre relativen Lagen zueinander.

Die Erfindung wird nachfolgend anhand von bevorzugten Ausführungsbeispielen beschrieben. Dabei offenbarte Merkmale bilden je einzeln und in jeder der Merkmalskombinationen die Gegenstände der Ansprüche vorteilhaft weiter. Es zeigen:
- Figur 1: eine erfindungsgemäße Vorrichtung in einem ersten Ausführungsbeispiel,
- Figur 2: einen Teil der Vorrichtung der Figur 1 in einem Längsschnitt,
- Figur 3: den Teil der Figur 2 in einer perspektivischen Ansicht,
- Figur 4: eine Blockiereinrichtung der Vorrichtung in einer Ansicht,
- Figur 5: die Blockiereinrichtung der Figur 4 in einer perspektivischen Ansicht,
- Figur 6: ein Blockierglied der Blockiereinrichtung in einem Querschnitt,
- Figur 7: eine erfindungsgemäße Vorrichtung in einem zweiten Ausführungsbeispiel,
- Figur 8: die Blockiereinrichtung der Vorrichtung nach Figur 7 und
- Figur 9: eine Weiterentwicklung der Vorrichtung nach Figur 1.

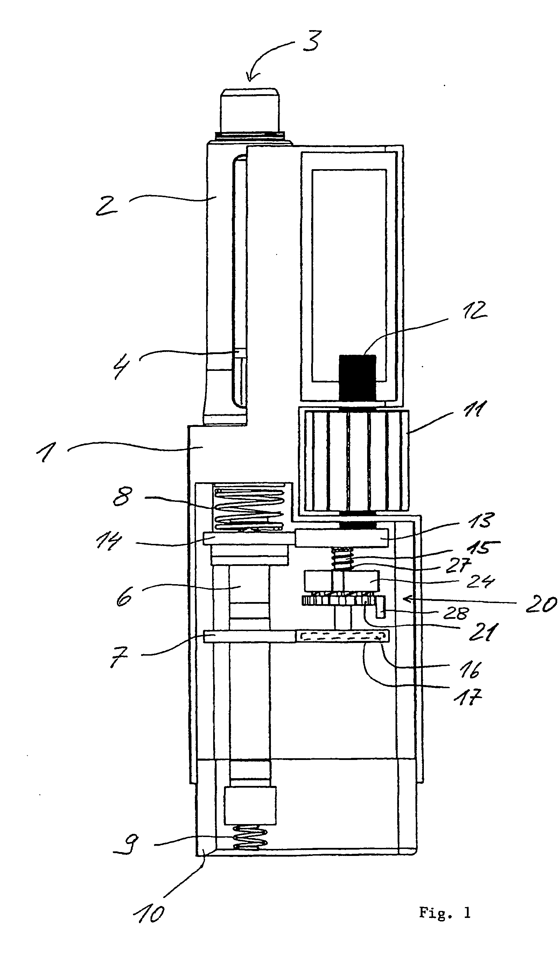

Figur 1 zeigt eine Vorrichtung zur dosierten Verabreichung eines injizierbaren Produkts, die als sogenannter Injektionspen ausgebildet ist. Der Pen weist ein Gehäuse mit einem vorderen Gehäuseteil 1 mit einer Aufnahme 2 für ein das Produkt enthaltendes Behältnis auf. Das Behältnis wird vorzugsweise durch eine Ampulle gebildet, in der ein Kolben verschiebbar angeordnet ist. Das Behältnis wird in die Aufnahme 2 eingesetzt und weist dann mit einem Behältnisauslass zu einem bei einer Injektion der Injektionsstelle zugewandten, proximalen Auslass 3 der Aufnahme 2. Durch Vorschieben des Kolbens auf den Auslass 3 zu wird das Produkt, beispielsweise Insulin, aus dem Behältnis verdrängt und durch einen angeschlossenen Katheter oder eine über den Auslass 3 unmittelbar von der Aufnahme 2 vorragende Inj ektionsnadel, auch bei 31G und grösser ausgeschüttet.

Um den Kolben in dem Behältnis auf den Auslass 3 zu vorzuschieben, wird ein Abtriebsglied 4 gegen die Rückseite des Kolbens gedrückt. Das Abtriebsglied 4 erfüllt die Funktion einer Kolbenstange und führt bei einer Ausschüttung des Produkts die gleiche Ausschüttbewegung wie der Kolben aus. Das Abtriebsglied 4 ist mit weiteren Komponenten an einem hinteren Gehäuseteil 10 gehalten, der von dem vorderen Gehäuseteil 1 hin und her geradverschiebbar aufgenommen wird und mit einem distalen Ende aus dem Gehäuseteil 1 herausragt. Durch Druck gegen das distale Ende des hinteren Gehäuseteils 10 wird das Abtriebsglied 4 auf den Auslass 3 zu vorgeschoben bis es zunächst an den Kolben anstößt und bei seiner weiteren Vorwärtsbewegung den Kolben im Behältnis vorschiebt. Diese Ausschüttbewegung des Abtriebsglieds 4 wird durch einen mechanischen Anschlag zwischen dem vorderen Gehäuseteil 1 und dem hinteren Gehäuseteil 10 in die proximale Richtung begrenzt. Der hintere Gehäuseteil 10 wird im folgenden seiner Funktion wegen auch als Betätigungseinrichtung bezeichnet. Bei der Ausschüttbewegung wird eine Druckfeder 8 gespannt. Nachdem der Druck von der Betätigungseinrichtung 10 genommen worden ist, schiebt die gespannte Druckfeder 8 die Betätigungseinrichtung 10 mit dem Abtriebsglied 4 wieder zurück in eine distale Endposition. In der distalen Endposition wird die auszuschüttende Produktdosis gewählt, indem ein lichter Abstand zwischen dem Abtriebsglied 4 und dem Kolben eingestellt wird. Bei der Einstellung des Abstands, d.h. der Dosierung, wird das Abtriebsglied 4 relativ zu der Betätigungseinrichtung 10 auf den Kolben zu bewegt.

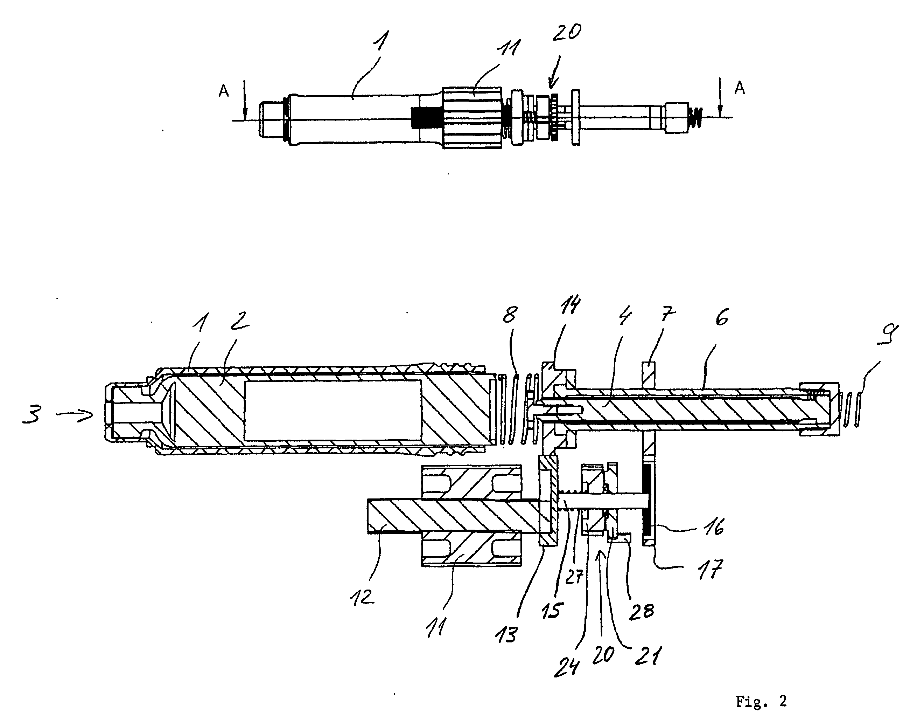

Wie in Figur 2 zu erkennen ist, erfolgt die Dosierung mittels eines Spindeltriebs. Das Abtriebsglied 4 ist die Ausgangsstufe bzw. Abtriebsstufe einer Antriebseinheit, die von einem für den Zweck der Dosierung einstufigen Spindeltrieb gebildet wird. Ein Stützglied 6 bildet die Abstützstufe des Spindeltriebs. Das Stützglied 6 wird durch eine Hülse mit einem Innengewinde an einem proximalen Ende gebildet. Das Abtriebsglied 4 ist eine Gewindestange mit einem sich über nahezu die gesamte Länge erstreckenden Außengewinde. Das Stützglied 6 umgibt das Abtriebsglied 4 koaxial. Das Außengewinde des Abtriebsglieds 4 und das Innengewinde des Stützglieds 6 greifen ineinander. Das Stützglied 6 ist zwischen der Druckfeder 8 und einer distalen Ausgleichsfeder 9 angeordnet. Die Ausgleichsfeder 9 bewirkt einen Längenausgleich; allerdings stößt das Stützglied 6 bei der Ausschüttbewegung mit seinem distalen Ende gegen die Betätigungseinrichtung 10, so dass trotz Ausgleichsfeder 9 eine Ausschüttbewegung von vorbestimmter Länge durchgeführt wird.

Neben der Achse der Ausschüttbewegung ist eine Dosiereinrichtung mit einer Blockiereinrichtung 20 angeordnet. Die Dosiereinrichtung umfasst einen Dosierknopf 11, der relativ zu den Gehäuseteilen 1 und 10, um eine zu der Achse der Ausschüttbewegung parallele Achse drehbar ist. Mit dem Dosierknopf 11 stellt der Verwender die auszuschüttende Produktdosis ein. Zentral durch den Dosierknopf 11 erstreckt sich eine Welle 12, die an ihrem Außenmantel mit einer Verzahnung versehen ist. Der Dosierknopf 11 weist eine Innenverzahnung auf, die in die Außenverzahnung der Welle 12 eingreift, so dass zwischen dem Dosierknopf 11 und der Welle 12 eine verdrehsichere Verbindung hergestellt wird. Allerdings ist die Welle 12 relativ zu dem Dosierknopf 11 in Wellenlängsrichtung hin und her verschiebbar. An ihrem distalen Ende kämmt die Welle 12 mit einem innenverzahnten Dosierglied 13. Das Zähnezahnverhältnis zwischen der Außenverzahnung der Welle 12 und der Innenverzahnung des Dosierglieds 13 beträgt 1:2. Das Dosierglied 13 weist ferner an seinem Außenmantel eine Außenverzahnung auf. Die Außenverzahnung des Dosierglieds 13 steht in einem kämmenden Zahneingriff mit einer Außenverzahnung eines weiteren Dosierglieds 14, das die Drehbewegung unmittelbar auf die Antriebseinheit, im Ausführungsbeispiel auf deren Abtriebsglied 4, überträgt und daher im folgenden als Übertragungsglied 14 bezeichnet wird.

Das Übertragungsglied 14 umgibt das Abtriebsglied 4 koaxial. Das Abtriebsglied 4 erstreckt sich durch das Übertragungsglied 14 und wird von dem Übertragungsglied 14 geführt, so dass das Abtriebsglied 4 relativ zu dem Übertragungsglied 14 in Längsrichtung verschoben, aber nicht um seine Längsachse verdreht werden kann. Das Übertragungsglied 14 ist an dem Stützglied 6 nicht verschiebbar, aber um die Längsachse verdrehbar befestigt. Bei einer Drehbewegung des Übertragungsglieds 14 um die gemeinsame Längsachse von Abtriebsglied 4 und Stützglied 6 wird daher das Abtriebsglied 4 mitgedreht und in Folge des Gewindeeingriffs mit dem Stützglied 6 relativ zu dem Stützglied 6 in Längsrichtung verschoben.

Voraussetzung dafür, dass das Abtriebsglied 4 durch Drehung des Übertragungsglieds 14 in Längsrichtung um eine definierte Weglänge relativ zu dem Stützglied 6 verschoben wird, ist allerdings, dass das Stützglied 6 seinerseits gegen ein Mitdrehen gesichert ist. Die Verdrehsicherung des Stützglieds 6 wird durch einen Zahneingriff mit einem Lagerkörper 17 bewirkt, der neben dem Stützglied 6 angeordnet ist. Der Lagerkörper 17 ist relativ zu dem Gehäuse 1,10 um eine Achse drehbar, die zu der Längsachse des Stützglieds 6 parallel ist. Im Ausführungsbeispiel fällt die Drehachse des Lagerkörpers 17 mit der Drehachse des Dosierglieds 13 zusammen, was zwar bevorzugt wird, aber nicht unumgänglich erforderlich ist. Der Lagerkörper 17 ist relativ zu dem Gehäuse 1,10 in der eingenommenen Drehwinkellage gesichert. Der Lagerkörper 17 ist als außenverzahntes Zahnrad ausgebildet und steht mit einem Zahnrad 7, welches das Stützglied 6 umgibt und verdrehgesichert mit dem Stützglied 6 verbunden ist, in Zahneingriff. Durch die Verdrehsicherung des Lagerkörpers 17 und den Zahneingriff mit dem Zahnrad 7 wird die Verdrehsicherung für das Stützglied 6 hergestellt.

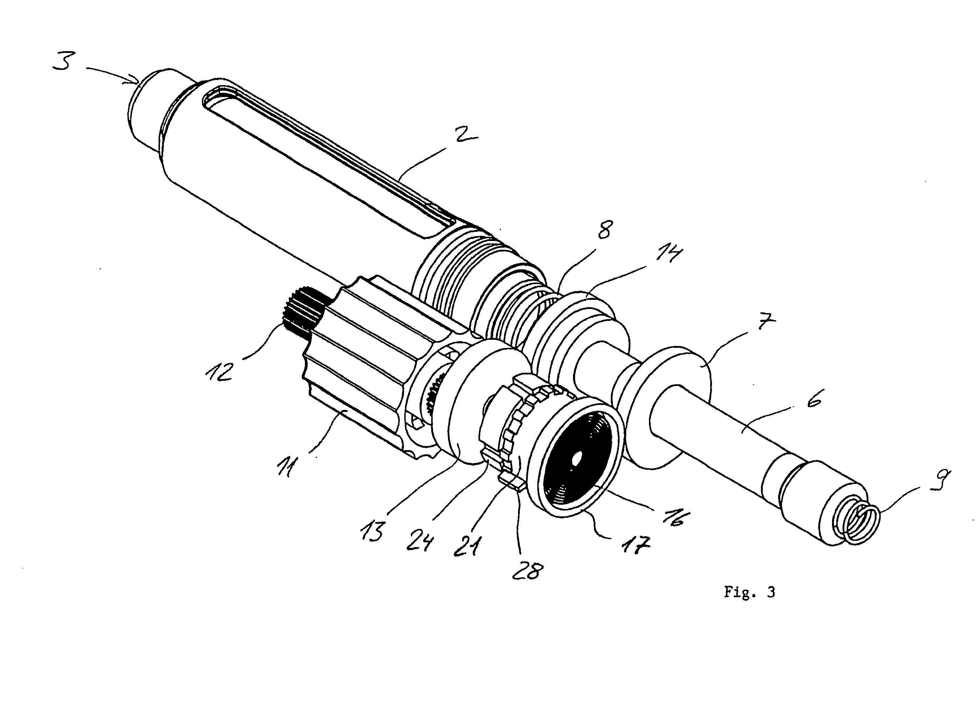

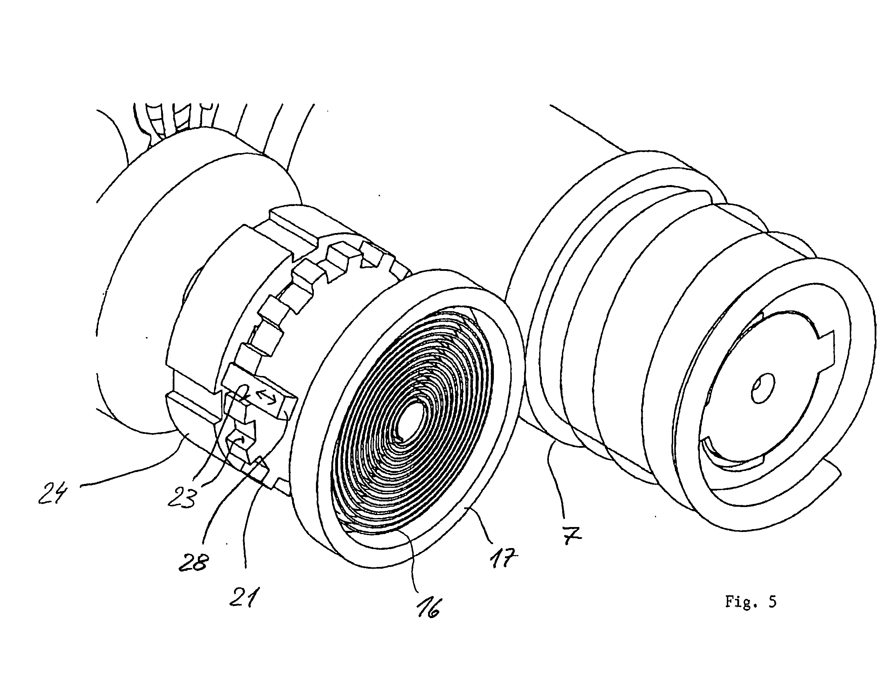

An dem Lagerkörper 17 ist eine Rücksetzfeder 16 abgestützt. Wie deutlicher in den Figuren 3 und 5 zu erkennen ist, wird die Rücksetzfeder 16 durch eine Spiralfeder mit spiralig einander umgebenden Federwindungen gebildet. Die Spiralfeder 16 ist mit einem äußeren Federende an dem Lagerkörper 17 und mit einem inneren Federende an einer Welle 15 abgestützt, die drehsteif mit dem Dosierglied 13 verbunden ist. Im Ausführungsbeispiel sind das Dosierglied 13 und die Welle 15 einstückig ausgebildet, wobei die Welle 15 zentrisch von dem Dosierglied 13 abragt und sich parallel zu der Längsachse des Abtriebsglieds 4 bis in den Lagerkörper 17 hinein erstreckt. Die Rücksetzfeder 16 ist sozusagen von ihrem inneren Federende um das distale Ende der Welle 15 spiralig bis zum äußeren Federende aufgewickelt. Hierbei ist die Rücksetzfeder 16 an der Welle 15 und dem Lagerkörper 17 derart abgestützt, dass bei einer Drehbewegung des Dosierglieds 13 relativ zu dem Lagerkörper 17 die Rücksetzfeder 16 je nach Drehrichtung entweder gespannt oder entspannt wird. Aufgrund der Kopplung zwischen der Antriebseinheit 4 und dem Dosierglied 13 sowie der Verdrehsicherung zwischen dem Lagerkörper 17 und dem Stützglied 6 oder allgemeiner noch, dem Gehäuse, ist die Rücksetzfeder 16 letztlich zwischen dem Abtriebsglied 4 und dem Stützglied 6 eingespannt, also zwischen denjenigen Gliedern, zwischen denen die Relativdrehung stattfindet, um das Abtriebsglied 4 aus einer Dosierendstellung in Richtung auf oder bis in eine Dosierausgangsstellung zurück zu bewegen. Als Dosierausgangsstellung wird hierin jede Stellung des Abtriebsglieds 4 relativ zu dem Kolben verstanden, aus der das Abtriebsglied 4 bei einer Ausschüttbewegung auf den Kolben zu bewegt, aber noch keine Ausschüttung stattfinden würde. Bei der Auswahl der auszuschüttenden Produktdosis, dem Dosieren, wird das Abtriebsglied 4 aus solch einer Dosierausgangsstellung heraus auf den Kolben zu bewegt und dadurch ein lichter Abstand zwischen dem Abtriebsglied 4 und dem Kolben verringert. Die Rücksetzfeder 16 ist nun so angeordnet, dass sie bei dieser Verstellbewegung der Antriebseinheit gespannt wird und sich bei einer Rückwärtsbewegung in Richtung auf die Dosierausgangsstellung zu entspannt.

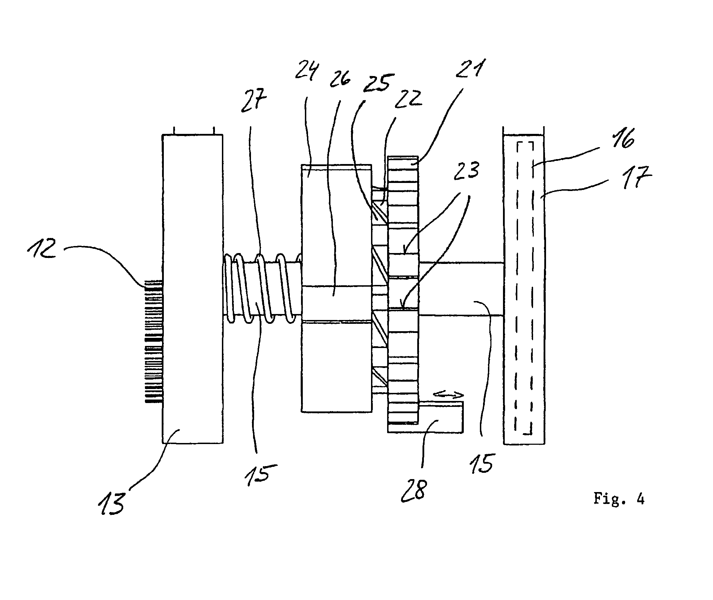

Figur 4 zeigt in einer vergrößerten Darstellung eine Blockiereinrichtung 20, die mit dem Dosierglied 13 verbunden ist. Durch die Blockiereinrichtung 20 wird die Federkraft der gespannten Rücksetzfeder 16 in Raststellungen der Blockiereinrichtung 20 von dem Dosierglied 13 und in Folge davon von dem Abtriebsglied 4 genommen.

Die Blockiereinrichtung 20 umfasst ein Blockierglied 21 und ein Blockiergegenglied 24. Das Blockierglied 21 ist ein scheibenförmiges Blockierrad, das verdrehgesichert mit dem Dosierglied 13 verbunden ist. Im Ausführungsbeispiel ist es verdrehgesichert auf der Dosiergliedwelle 15 befestigt. Das Blockiergegenglied 24 ist als Ringscheibe ausgebildet, durch die sich die Dosiergliedwelle 15 erstreckt. Das Blockiergegenglied 24 ist mit dem Gehäuseteil 10 verdrehgesichert verbunden und relativ zu dem Gehäuseteil 10 entlang der Dosiergliedwelle 15 hin- und her bewegbar geführt. Als Verdrehsicherung und Geradführung dienen Nuten 26, die sich am Außenmantel des Blockiergegenglieds 24 in Längsrichtung erstrecken und in die entsprechende Führungsvorsprünge des Gehäuseteils 10 eingreifen.

An Stirnseiten, die sich gegenüberliegen, weisen das Blockierglied 21 und das Blockiergegenglied 24 je über einen zur Dosiergliedwelle 15 konzentrischen Kreis gleichmäßig verteilt angeordnete Sägezähne 22 und 25 auf. Die Sägezähne 22 des Blockierglieds 21 und die Sägezähne 25 des Blockiergegenglieds 24 sind so angeordnet, dass die allmählich ansteigenden Flanken sämtlicher Sägezähne 22 stets gleichzeitig über die allmählich ansteigenden Flanken der Sägezähne 25 gleiten, wenn das Dosierglied 13 so gedreht wird, dass die Antriebseinheit in Richtung auf eine Dosierendstellung zu verstellt wird. Um die Gleitbewegung der Sägezähne 22 und 25 zu ermöglichen, ist das Blockiergegenglied 24 gegen die Rückstellkraft einer Stützfeder 27 von dem Blockierglied 21 wegdrückbar. Sobald die Sägezähne 22 und 25 mit ihren flachen Flanken übereinandergeglitten sind, schnappt das Blockiergegenglied 24 aufgrund der Federkraft der Stützfeder 27 wieder gegen das Blockierglied 21 vor. Eine Relativverdrehung zwischen dem Blockierglied 21 und dem Blockiergegenglied 24 in die Gegenrichtung wird dadurch verhindert, dass sich in Bezug auf die Gegenrichtung die steilen Flanken der Sägezähne 22 und 25 gegenüberliegen und dadurch ein Rückdrehen des Blockierglieds 21 verhindert wird. Ein Weiterdrehen des Blockierglieds 21 aus einer Raststellung in die nächste Raststellung entspricht einer einstellbaren Dosiseinheit.

Die Blockiereinrichtung 20 dient nicht nur dem gänzlichen Verhindern einer Rücksetzung der Antriebseinheit. Mit ihrer Hilfe kann im Falle einer Überdosierung die fälschlicherweise zu hoch eingestellte Dosis Einheit für Einheit wieder zurückgestellt werden. Bei dem Zurückstellen wirken ein Entsicherungselement 28 und Anschläge 23, die an dem Blockierglied 21 ausgebildet sind, zusammen.

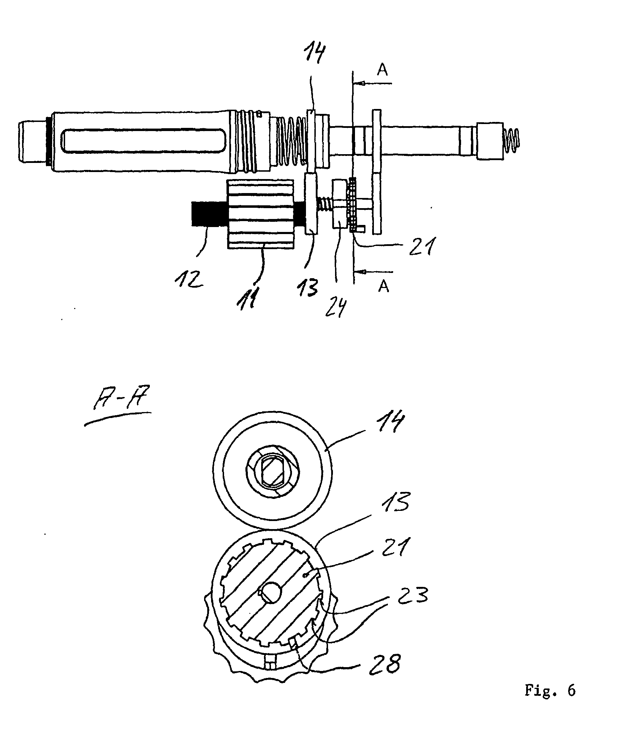

Wie den in Figuren 4 und 5 und dem Querschnitt A-A der Figur 6 zu erkennen ist, werden die Anschläge 23 durch einen Zackenkranz an dem Außenmantel des Blockierglieds 21 gebildet. Der Zackenkranz wird durch gerade Nuten gebildet, die in die Außenmantelfläche des Blockierglieds 21 eingelassen sind und sich axial von einer Stirnseite des Blockierglieds 21 bis zur anderen durchgehend erstrecken. Die Anschläge 23 werden je durch eine der beiden in Umfangsrichtung gesehen einander zugewandten Nutwände gebildet. Das Entsicherungselement 28 ist ein Schieber, der an dem Gehäuseteil 10 in die Nuten des Blockierglieds 21 in Richtung auf das Blockiergegenglied 24 zu einschiebbar und wieder aus den Nuten herausschiebbar angeordnet ist. Das Einschieben erfolgt gegen eine Federkraft, so dass das Entsicherungselement 28 nach einem Loslassen selbsttätig wieder aus dem Nuteingriff herausbewegt wird. Die Verschieberichtung ist in den Figuren 4 und 5 mit einem Doppelpfeil eingezeichnet. Das Entsicherungselement 28 weist eine in Umfangsrichtung des Blockierglieds 21 gemessene Breite auf, die kleiner ist als die Breite der Nuten des Blockierglieds 21. Nach einem Einfahren des Entsicherungselements 28 in eine der Nuten verbleibt zwischen dem am Blockierglied 21 gebildeten Anschlag 23 und der gegenüberliegenden Fläche des Entsicherungselements 28 ein lichter Abstand, wie dies am besten im Querschnitt der Figur 6 zu erkennen ist.

Im folgenden wird die Funktionsweise der Vorrichtung der ersten Ausführungsform erläutert:
In Figur 2 nimmt die Antriebseinheit ihre erste Dosierausgangsstellung vor einer ersten Ausschüttung und vor einer ersten Dosierung ein. Ein in der Aufnahme 2 aufgenommenes Behältnis ist noch vollkommen gefüllt. Aus dieser ersten Dosierausgangsstellung heraus wird die erste Dosierung vorgenommen. Hierfür wird der Dosierknopf 11 relativ zu dem Gehäuse 1, 10 (Figur 1) verdreht. Durch die Drehung des Dosierknopfs 11 wird die Welle 12 mitgedreht. Die Welle 12 kämmt mit der Innenverzahnung des Dosierglieds 13. Durch das Zähnezahnverhältnis wird die Drehwinkelgeschwindigkeit der Welle 12 bei der Übertragung auf das Dosierglied 13 im Verhältnis 2:1 untersetzt. Das Dosierglied 13 kämmt mit dem Übertragungsglied 14 im Verhältnis 1:1.

Das Übertragungsglied 14 nimmt bei seiner Drehung das verdrehgesichert verbundene Abtriebsglied 4 mit. Das Stützglied 6 wird aufgrund des Zahneingriffs zwischen dem Zahnrad 7 und dem Lagerkörper 17 und der Verdrehsicherung des Lagerkörpers 17 relativ zu dem Gehäuse 1,10 in seiner Drehwinkellage relativ zu dem Lagerkörper 17 und relativ zu dem Gehäuse 1,10 gehalten. In Folge des Gewindeeingriffs zwischen dem Abtriebsglied 4 und dem Stützglied 6 wird das sich drehende Abtriebsglied 4 auf den Auslass 3 zu bewegt.

Durch die Drehbewegung und die verdrehsichere Verbindung zwischen dem Dosierglied 13 und der Welle 15 wird zum einen die Rücksetzfeder 16 gespannt und zum anderen das Blockierglied 21 relativ zu dem Blockiergegenglied 24 mitgedreht. Die Relativdrehung zwischen dem Blockierglied 21 und dem Blockiergegenglied 24 wird durch den Sägezahneingriff und die federnde Hin- und Herbewegbarkeit des Blockiergegenglieds 24 ermöglicht. Aufgrund des Sägezahneingriffs wird jedoch eine Rückdrehung verhindert. Durch den Sägezahneingriff wird ferner ein gut hörbares Klick-Geräusch erzeugt, das dem Verwender jeweils die Erhöhung um eine Dosiseinheit zu Gehör bringt. Zusätzlich ist vorzugsweise eine optische Anzeige der Anzahl der bei dieser Dosierung kumuliert gewählten Dosiseinheiten vorgesehen, die auf einer elektronischen Erfassung der Drehwinkellage eines der bei der Dosierung verdrehten Glieder beruht.

Nach dem Auswählen der Dosis wird das Stützglied 6 mit dem Abtriebsglied 4 zusammen mit den weiteren Bestandteilen der Dosiereinrichtung einschließlich der Blockiereinrichtung 20 durch manuellen Druck gegen die Betätigungseinrichtung 10 relativ zu dem Gehäuseteil 1 auf den Auslass 3. zu bis in die proximale Endposition der Betätigungseinrichtung 10 vorgeschoben. Im Zuge dieser Ausschüttbewegung stößt das Abtriebsglied 4 zunächst gegen den Kolben, der bei dem weiteren Vorschieben des Abtriebsglieds 4 im Behältnis auf den Auslass 3 zu vorgeschoben wird. Aufgrund des Vorschiebens des Kolbens wird eine der eingestellten Dosis entsprechende Produktmenge aus dem Behältnis verdrängt und durch den Auslass 3 sowie eine angeschlossene Injektionsnadel ausgeschüttet. Sobald der Druck von der Betätigungseinrichtung 10 weggenommen wird, bewegt sich die Betätigungseinrichtung 10 unter den Druck der Druckfeder 8 wieder zurück in ihre distale Endposition. In der distalen Endposition der Betätigungseinrichtung 10 nimmt das Abtriebsglied 4 nunmehr seine neue Dosierausgangsstellung für eine erneute Dosierung und Produktausschüttung ein. Dieser Vorgang, d.h. die Dosierung und die anschließende Ausschüttbewegung, können mehrmals wiederholt werden, bis das Abtriebsglied 4 nach Erreichen seiner letzten Dosierendstellung die Ausschüttbewegung ausgeführt hat und das Behältnis idealerweise vollkommen entleert ist.

Am Ende der Ausschüttbewegung stößt die Welle 12 gegen einen mit einer optischen Anzeige verbundenen Schalter, wodurch die Anzeige auf "0" zurückgesetzt wird. Der Eingriff zwischen dem Dosierglied 13 und der Welle 12 ist entsprechend verschiebegesichert, so dass die Welle 12 sämtliche Verschiebebewegungen des Dosierglieds 13 mitmacht.

Für eine erneute Verwendung der Vorrichtung muss das Abtriebsglied 4 aus der letzten Dosierendstellung zurückbewegt werden, vorzugsweise bis in seine erste Dosierausgangsstellung. Die Rücksetzbewegung wird mittels der Rücksetzfeder 16 automatisch ausgeführt, wenn die Verdrehsicherung zwischen dem Lagerkörper 17 und dem hinteren Gehäuseteil 10 gelöst wird. Hierfür ist ein eigenes Entsicherungselement vorgesehen, beispielsweise ein weiterer Schieber, der zum Herstellen der Verdrehsicherung in den Lagerkörper 17 eingreift und bei dem Lösen aus den Eingriff mit dem Lagerkörper 17 herausbewegt wird. Nach dem Lösen der Verdrehsicherung dreht der Lagerkörper 17 aufgrund der gespeicherten Federenergie um eine Drehachse, die mit derjenigen des Dosierglieds 13 zusammenfällt. Aufgrund des Stirneingriffs mit dem Lagerkörper 17 werden das Zahnrad 7 und das damit verdrehgesichert verbundene Stützglied 6 zwangsweise mit verdreht. Durch den Blockiereingriff des Blockierglieds 21 mit dem Blockiergegenglied 24 wird das Dosierglied 13 in der eingenommenen Drehwinkellage relativ zu dem hinteren Gehäuseteil 10 gesichert. Durch den Zahneingriff behalten auch das Übertragungsglied 14 und das damit verdrehgesichert verbundene Abtriebsglied 4 ihre eingenommenen Drehwinkelstellungen. Durch die auf diese Weise erzwungene Relativdrehung zwischen dem Stützglied 6 und dem Abtriebsglied 4 wird das Abtriebsglied 4 in das Stützglied 6 bis in seine erste Dosierausgangsstellung eingefahren. Die Rücksetzfeder 16 ist in dieser ersten Dosierausgangsstellung vorzugsweise vorgespannt, um ein vollständiges Zurückfahren des Abtriebsglieds 4 auch nach mehrmaliger Verwendung der Vorrichtung sicher zu stellen.

Falls der Verwender bei der Dosierung, d.h. der Verstellbewegung der Antriebseinheit aus einer Dosierausgangsstellung in die nächste Dosierendstellung, versehentlich zuviel Dosiseinheiten gewählt hat, kann er diese Überdosierung mittels des Entsicherungselements 28 korrigieren. Für die Korrektur wird das Entsicherungselement 28 gegen den Druck einer Feder in die axial gegenüberliegende Nut des Blockierglieds 21 hineingeschoben und bis gegen das Blockiergegenglied 24 weiter geschoben, bis das Blockiergegenglied 24 gegen den Druck der Stützfeder 27 von dem Blockierglied 21 abhebt. Das Entsicherungselement 28 ist soweit gegen das Blockiergegenglied 24 vorschiebbar, dass die in der eingenommenen Raststellung einander gegenüberliegenden steilen Flanken der Sägezähne 22 und 25 (Figur 4) außer Eingriff gelangen. Sobald dieser Blockiereingriff gelöst ist, dreht das Blockiergegenglied 24 aufgrund der Federkraft der Rücksetzfeder 16 entgegen der Drehrichtung bei Erhöhung der Dosis. Die Rückdrehung des Blockierglieds 21 wird jedoch durch das Entsicherungselement 28 begrenzt. Das Blockierglied 21 kann sich nämlich nur um den lichten Abstand zurückdrehen, der zwischen dem Entsicherungselement 28 und dem durch die gegenüberliegende Nutwand gebildeten Anschlag 23 verbleibt. Wird das Entsicherungselement 28 losgelassen, so fährt es aufgrund der auf ihm lastenden Federkraft wieder zurück und schließlich aus der Nut des Blockierglieds 21 heraus. Bei dieser Bewegung wird zunächst das Blockiergegenglied 24 von der Stützfeder 27 wieder gegen das Blockierglied 21 gedrückt. Sobald das Entsicherungselement 28 vollkommen aus der Nut des Blockierglieds 21 herausgefahren und das Anschlagpaar 23, 28 aufgelöst worden sind, dreht das Blockierglied 21 unter der Federkraft der Rücksetzfeder 16 weiter zurück, bis die einander gegenüberliegenden steilen Zahnflanken der Sägezähne 22 und 25 aneinander stoßen. Die Rückdrehung des Blockierglieds 21 entspricht bei einer Betätigung des Entsicherungselements 28 dem Zurücksetzen um eine Dosiseinheit.

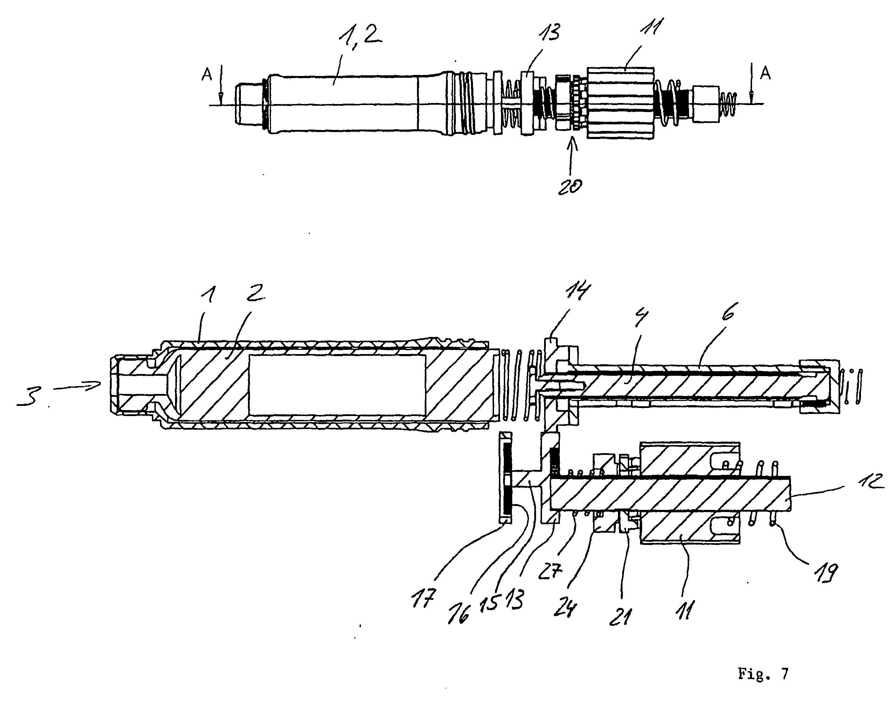

Figur 7 zeigt in einem Längsschnitt die wesentlichen Teile einer Vorrichtung, nach einem zweiten Ausführungsbeispiel. In diesem Ausführungsbeispiel werden beide Verstellbewegungen der Antriebseinheit, nämlich die Bewegung des Abtriebsglieds 4 in Richtung auf die Dosierendstellung und die Bewegung in Richtung auf die Dosierausgangsstellung zu, durch den Eingriff mit dem Dosierglied 13 bewirkt. Der Lagerkörper 17 ist einfach verdrehgesichert mit dem hinteren Gehäuseteil 10 verbunden und mit dem Spindeltrieb nur über das Dosierglied 13 gekoppelt. Die Rücksetzfeder 16 ist mit einem inneren Ende auf einer zentrisch von dem Dosierglied 13 abragenden und verdrehgesichert damit verbundenen Welle oder Zapfen 15 abgestützt. Das Stützglied 6 wird verdrehgesichert unmittelbar von dem Gehäuseteil 1 (Fig. 1) gehalten. In Bezug auf die Antriebseinheit und die Einspannung der Rücksetzfeder 16 ergibt sich keine weitere Änderung zu der Vorrichtung des ersten Ausführungsbeispiels. Im zweiten Ausführungsbeispiel kann der Spindeltrieb jedoch alternativ zwischen dem Abtriebsglied 4 und dem Übertragungsglied 14 gebildet werden und das Stützglied 6 bei der Verstellbewegung als reine Geradführung für das Abtriebsglied 4 dienen.

Die Blockiereinrichtung 20 ist jedoch nicht auf einer verdrehgesichert mit dem Dosierglied 13 verbundenen Welle, sondern entlang der Dosierwelle 12 angeordnet. Der Eingriff zwischen der Welle 12 und dem Dosierglied 13 ist allerdings der gleiche wie bei der Vorrichtung des ersten Ausführungsbeispiels.

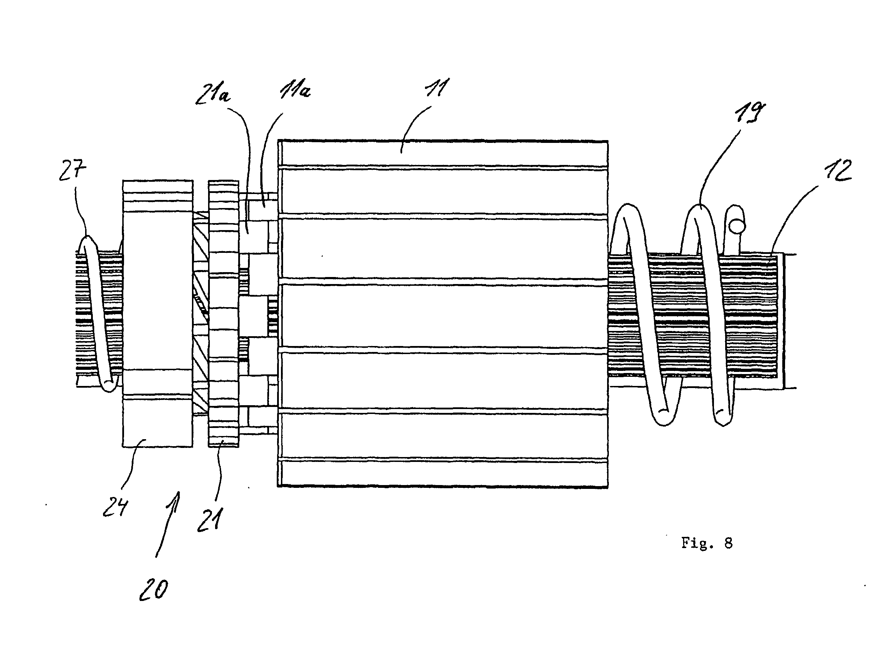

Figur 8 zeigt die Blockiereinrichtung 20 in einer vergrößerten Darstellung. Das Blockierglied 21 umgibt die Welle 12 und ist relativ zu der Welle 12 verdrehbar gelagert. Der Dosierknopf 11 ist wie in der ersten Ausführungsform verdrehgesichert mit der Welle 12 verbunden. An ihren einander zugewandten Stirnseiten weisen der Dosierknopf 11 und das Blockierglied 21 eine Mehrzahl von Klauen 11a und 21 a auf, die zur Ausbildung einer Verdrehsicherung zwischen dem Dosierknopf 11 und dem Blockierglied 21 ineinander greifen, wie dies in Figur 8 dargestellt ist. Der Dosierknopf 11 kann gegen die Kraft einer Feder 19 von dem Blockierglied 21 weg aus dem durch die Klauen 11a und 21 gebildeten Verdrehsicherungseingriff bewegt werden. Das Blockiergegenglied 24 ist wieder verdrehgesichert, aber entlang der Welle 12 gegen die Kraft einer Stützfeder 27 auf das Dosierglied 13 zu geradverschiebbar gelagert. Die Ausbildung und Funktionsweise des Blockierglieds 21 und des Blockiergegenglieds 24 ist mit Ausnahme der Drehmitnahme des Blockierglieds 21 durch den Drehknopf 11 die Gleiche wie in dem ersten Ausführungsbeispiel.

Auch in Bezug auf die Funktionsweise kann mit Ausnahme der vollständigen Rücksetzung der Antriebseinheit in seine erste bzw. distalste Dosierausgangsstellung vollinhaltlich auf die Ausführungen zu dem ersten Ausführungsbeispiel verwiesen werden. Um jedoch nach Erreichen der letzten Dosierendstellung die Rücksetzbewegung auszulösen, wird der Dosierknopf 11 gegen die Kraft einer Feder 19 von dem Blockierglied 21 weggeschoben, wodurch die Verdrehsicherung zwischen dem Dosierknopf 11 und dem Blockierglied 21 gelöst wird. Nach dem Lösen der Verdrehsicherung werden die Welle 12, der Dosierknopf 11 und das Dosierglied 13 durch die Federkraft der Rücksetzfeder 16 zurückgedreht. Über den Eingriff mit dem Übertragungsglied 14 wird bei diesem Zurückdrehen das Abtriebsglied 4 in seine distalste Dosierausgangsstellung zurückbewegt.

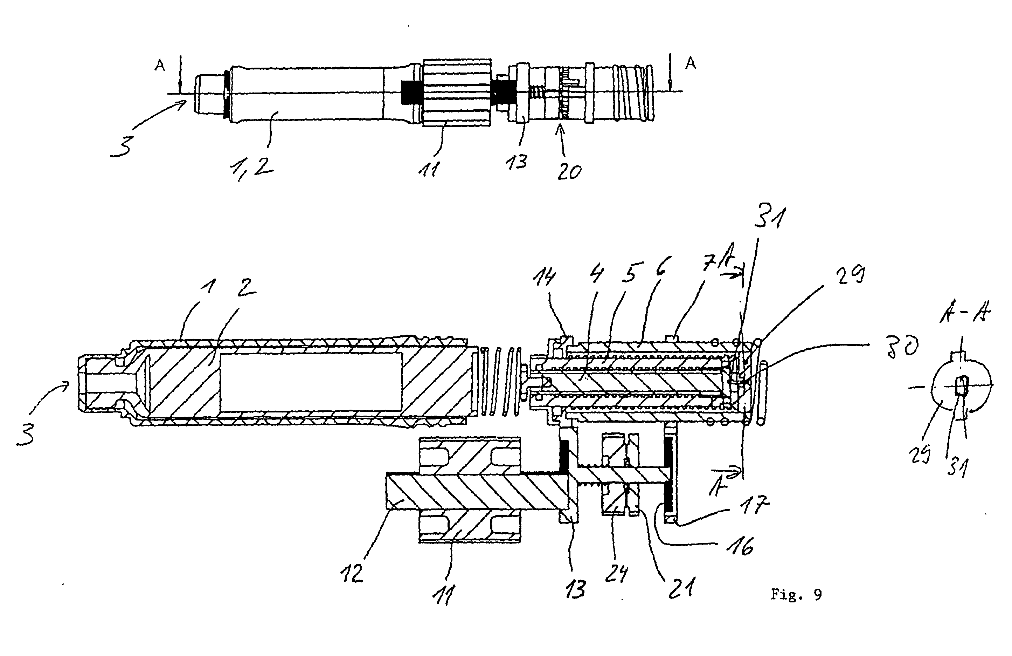

Figur 9 zeigt ein drittes Ausführungsbeispiel, das sich von dem ersten Ausführungsbeispiel lediglich in Bezug auf die Antriebseinheit unterscheidet. Der Spindeltrieb der Antriebseinheit des dritten Ausführungsbeispiels ist zweistufig mit dem Abtriebsglied 4 als Ausgangsstufe, einem Antriebsglied 5 als Zwischenstufe und dem Stützglied 6 als Abstützstufe. Das Stützglied 6 wird wie bei den beiden anderen Ausführungsbeispielen während der Dosierung relativ zu dem hinteren Gehäuseteil 10 verdrehgesichert und verschiebegesichert gehalten.

Das von dem Dosierglied 13 drehangetriebene Übertragungsglied 14 nimmt bei seiner Drehbewegung das Antriebsglied 5 mit. Dabei wird das Antriebsglied 5 durch einen Gewindeeingriff mit dem Stützglied 6 relativ zu dem Stützglied 6 in Richtung auf den Auslass 3 zu vorbewegt. Das Antriebsglied 5 nimmt bei seiner Translationsbewegung das Abtriebsglied 4 mit. Zusätzlich wird das Abtriebsglied 4 durch einen Gewindeeingriff mit dem Antriebsglied 5 relativ zu dem Antriebsglied 5 in Richtung auf den Auslass 3 zu vorbewegt. Um die Bewegungen des Antriebsglieds 5 und des Abtriebsglieds 4 relativ zueinander und relativ zu dem Stützglied 6 zu erhalten, ist an dem distalen Ende des Antriebsglieds 5 eine Verdrehsicherung 29 in Form einer Scheibe befestigt. Die Befestigung ist derart, dass das Antriebsglied 5 relativ zu der Verdrehsicherung 29 drehbar ist, die Verdrehsicherung 29 aber bei seiner eigenen Tranlationsbewegung relativ zu dem Stützglied 6 mitnimmt. Die Verdrehsicherung 29 ist mit dem Stützglied 6 verdrehgesichert verbunden, beispielsweise wie im Schnitt A-A dargestellt durch Eingriff von zwei Vorsprüngen in entsprechende Längsnuten des Stützglieds 6. An der Verdrehsicherung 29 ist ein stiftförmiger Mitnehmer 31 verdrehgesichert befestigt, beispielsweise wie im Ausführungsbeispiel mit einer Schraube 30. Das Abtriebsglied 4 steht mit dem Mitnehmer 31 derart in Eingriff, dass eine Drehung des Abtriebsglieds relativ zu dem Mitnehmer 31 verhindert wird, aber eine Translationsbewegung des Abtriebsglieds 4 relativ zu dem Mitnehmer 31 möglich ist. Das Abtriebsglied 4 wird somit über dem Mitnehmer 31 und die Verdrehsicherung 29 mit dem Stützglied 6 verdrehgesichert verbunden. Durch die Mehrstufigkeit mit mehreren in Längsrichtung bewegten Stufen 4 und 5 wird ein in Längsrichtung kurz bauender Spindeltrieb mit einer großen Dosierlänge erhalten.

### Bezugszeichen

- 1: Gehäuseteil
- 2: Behältnisaufnahme
- 3: Auslass
- 4: Abtriebsglied
- 5: Antriebsglied
- 6: Stützglied
- 7: Zahnrad
- 8: Druckfeder
- 9: Ausgleichsfeder
- 10: Gehäuseteil, Betätigungseinrichtung
- 11: Dosierknopf
- 11a: Klauen
- 12: Welle
- 13: Dosierglied, Drehglied
- 14: Dosierglied, Übertragungsglied
- 15: Welle
- 16: Rücksetzfeder
- 17: Lagerkörper
- 18: -
- 19: Feder
- 20: Blockiereinrichtung
- 21: Blockierglied
- 21a: Klauen
- 22: Sägezähne
- 23: Anschlag
- 24: Blockiergegenglied
- 25: Sägezähne
- 26: Geradführung
- 27: Stützfeder
- 28: Entsicherungselement
- 29: Verdrehsicherung
- 30: Schraube
- 31: Mitnehmer

## Patentansprüche

1. Vorrichtung zur dosierten Verabreichung eines injizierbaren Produkts, die Vorrichtung umfassend:
a) ein Gehäuse (1,10) mit einer Aufnahme (2) für ein Behältnis, welches das Produkt enthält und für eine Ausschüttung einer ausgewählten Produktdosis einen Kolben in Richtung auf einen Auslass (3) vorschiebbar aufnimmt,
b) ein Dosierglied (13), mit dem für ein Auswählen der Produktdosis eine Dosierbewegung relativ zu dem Gehäuse (1, 10) ausführbar ist,
c) eine Antriebseinheit (4, 6; 4, 5, 6), die mit dem Gehäuse (1,10) und dem Dosierglied (13) derart gekoppelt ist, dass durch die Dosierbewegung des Dosierglieds (13) die Antriebseinheit (4, 6; 4, 5, 6) aus einer Dosierausgangsstelle in eine Dosierendstellung relativ zu dem Gehäuse (1,10) verstellbar ist,
d) und eine Betätigungseinrichtung (10), welche die Antriebseinheit (4, 6; 4, 5, 6) veranlasst, eine Ausschüttbewegung auszuführen, durch die der Kolben in Richtung auf den Auslass (3) zu vorgeschoben wird,
**dadurch gekennzeichnet, dass**
e) eine Rücksetzfeder (16), die in einem gespannten Zustand gesichert ist, durch Entsicherung mit der Antriebseinheit (4, 6; 4, 5, 6) gekoppelt wird und eine Rücksetzbewegung der Antriebseinheit (4, 6; 4, 5, 6) in Richtung auf ihre Dosierausgangsstellung zu bewirkt.

2. Vorrichtung nach Anspruch 1, **dadurch gekennzeichnet, dass** die Rücksetzfeder (16) durch eine in Richtung auf die Dosierendstellung erfolgende Verstellung der Antriebseinheit (4, 6; 4, 5, 6) gespannt wird und eine Blockiereinrichtung (20) die Rücksetzbewegung der Antriebseinheit (4, 6; 4, 5, 6) verhindert.

3. Vorrichtung nach einem der vorhergehenden Ansprüche, **dadurch gekennzeichnet, dass** eine Spiralfeder, die zwischen dem Gehäuse (1,10) und einem relativ zu dem Gehäuse (1,10) drehbaren Drehglied (13) eingespannt ist, die Rücksetzfeder (16) bildet, wobei das Drehglied (13) bei der Verstellung der Antriebseinheit (4, 6; 4, 5, 6) gedreht und dadurch die Rücksetzfeder (16) gespannt wird.

4. Vorrichtung nach einem der vorhergehenden Ansprüche, **dadurch gekennzeichnet, dass** das Dosierglied (13) bei der Dosierbewegung über einen Spindeltrieb ein Abtriebsglied (4) der Antriebseinheit (4, 6; 4, 5, 6) in Richtung auf den Kolben zu vorbewegt.

5. Vorrichtung nach dem vorhergehenden Anspruch, **dadurch gekennzeichnet, dass** der Spindeltrieb ein Stützglied (6) umfasst, das relativ zu dem Gehäuse (1,10) drehbar gelagert ist, aber gegen eine Verdrehung lösbar gesichert wird.

6. Vorrichtung nach einem der vorhergehenden Ansprüche, **dadurch gekennzeichnet, dass**
eine Blockiereinrichtung (20) in einem Blockiereingriff die Rücksetzbewegung der Antriebseinheit (4, 6; 4, 5, 6) verhindert,
die Rücksetzfeder (16) zwischen einem Lagerkörper (17) und einem Drehglied (13) eingespannt ist,
wobei das Drehglied (13) bei der Verstellung der Antriebseinheit (4, 6; 4, 5, 6) gedreht wird,
und wobei der Lagerkörper (17) relativ zu dem Gehäuse (1,10) drehbar gelagert ist und mit der Antriebseinheit (4, 6; 4, 5, 6) in einem Eingriff für einen Drehantrieb steht,
und dass eine Verdrehsicherung, die eine Drehbewegung des Lagerkörpers (17) relativ zu dem Gehäuse (1,10) verhindert, lösbar ist.

7. Vorrichtung nach einem der vorhergehenden Ansprüche, **dadurch gekennzeichnet, dass** ein mit der Antriebseinheit (4, 6; 4, 5, 6) gekoppeltes Blockierglied (21) und ein mit dem Gehäuse (1,10) gekoppeltes Blockiergegenglied (24) in einem Blockiereingriff die Rücksetzbewegung der Antriebseinheit (4, 6; 4, 5, 6) verhindern.

8. Vorrichtung nach dem vorhergehenden Anspruch, **dadurch gekennzeichnet, dass** das Blockierglied (21) zur Bildung des Blockiereingriffs in Raststellungen mit dem Blockiergegenglied (24) verrastbar ist und ein Entsicherungselement (28) vorgesehen ist, um den Blockiereingriff zu lösen.

9. Vorrichtung nach dem vorhergehenden Anspruch, **dadurch gekennzeichnet, dass** das Blockierglied (21) einen Anschlag (23) ausbildet, der nach einem Lösen des Blockiereingriffs gegen das Entsicherungselement (28) zu liegen kommt und die Rücksetzbewegung der Antriebseinheit (4, 6; 4, 5, 6) begrenzt.

10. Vorrichtung nach einem der drei vorhergehenden Ansprüche, **dadurch gekennzeichnet, dass** das Blockierglied (21) und das Blockiergegenglied (24) in dem Blockiereingriff mittels einer Feder (27) gegeneinander gedrückt werden.

11. Vorrichtung nach einem der vier vorhergehenden Ansprüche, **dadurch gekennzeichnet, dass**
die Dosierbewegung des Dosierglieds (13) eine Drehbewegung ist,
mit dem Dosierglied (13) eine Welle (15;12) verbunden ist, die sich bei einer Drehbewegung des Drehglieds (13) ebenfalls dreht,
das Blockierglied (21) verdrehgesichert auf der Welle (15;12) sitzt und
das Blockiergegenglied (24) längs der Welle (15; 12) gegen die Kraft einer Feder (27) aus dem Blockiereingriff bringbar ist.

12. Vorrichtung nach einem der vorhergehenden Ansprüche, **dadurch gekennzeichnet, dass** das Dosierglied (13) relativ zu dem Gehäuse (1,10) um eine Drehachse drehbar gelagert ist und von dem Dosierglied (13) in Richtung der Drehachse eine Welle (15) abragt, an welcher die Rücksetzfeder (16) abgestützt ist.

13. Vorrichtung nach einem der vorhergehenden Ansprüche, **dadurch gekennzeichnet, dass**
ein Dosierknopf (11) von dem Gehäuse (1,10) drehbar gelagert wird,
der Dosierknopf (11) mit einer Welle (12) derart in Eingriff steht, dass eine Drehbewegung des Dosierknopfs (11) eine Drehbewegung der Welle (12) bewirkt und eine Längsverschiebung der Welle (12) relativ zu dem Dosierknopf (11) möglich ist,
und die Welle (12) mit dem Dosierglied (13) in einem Eingriff steht, durch den die Dosierbewegung des Dosierglieds (13) erzeugt wird.

14. Vorrichtung nach einem der vorhergehenden Ansprüche, **dadurch gekennzeichnet, dass** das Dosierglied (13) und die Rücksetzfeder (16) neben der Antriebseinheit (4, 6; 4, 5, 6) in dem Gehäuse (1,10) angeordnet sind.

## Claims

1. A device for the dosaged administration of an injectable product, the device including:
a) a casing (1, 10) having a receiving means (2) for a container which contains the product and which accommodates a piston which can be advanced in a direction towards an outlet (3) for expelling a selected dose of product,
b) a dosing member (13) with which a dosing movement relative to the casing (1, 10) can be effected for selecting the dose of product,
c) a drive unit (4, 6; 4, 5, 6) which is coupled to the casing (1, 10) and the dosing member (13) in such a way that the drive unit (4, 6; 4, 5, 6) is displaceable from an initial dosing position to a final dosing position relative to the casing (1, 10) by the dosing movement of the dosing member (13), and
d) an actuating device (10) which causes the drive unit (4, 6; 4, 5, 6) to perform an expelling movement by which the piston is advanced towards the outlet (3),
**characterised in that**
e) a resetting spring (16) which is secured in a stressed state is coupled by being released to the drive unit (4, 6; 4, 5, 6) and causes a resetting movement of the drive unit (4, 6; 4, 5, 6) in a direction towards its initial dosing position.

2. A device according to claim 1 **characterised in that** the resetting spring (16) is stressed by displacement of the drive unit (4, 6; 4, 5, 6), which takes place in a direction towards the final dosing position, and a blocking device (20) prevents the resetting movement of the drive unit (4, 6; 4, 5, 6).

3. A device according to one of the preceding claims **characterised in that** a coil spring which is stressed between the casing (1, 10) and a rotary member (13) which is rotatable relative to the casing (1, 10) forms the resetting spring (16), wherein the rotary member (13) is rotated upon displacement of the drive unit (4, 6; 4, 5, 6) and the resetting spring (16) is stressed thereby.

4. A device according to one of the preceding claims **characterised in that** the dosing member (13) advances a drive output member (4) of the drive unit (4, 6; 4, 5, 6) in a direction towards the piston in the dosing movement by way of a spindle drive.

5. A device according to the preceding claim **characterised in that** the spindle drive includes a support member (6) which is mounted rotatably relative to the casing (1, 10) but which is releasably secured to prevent rotational movement.

6. A device according to one of the preceding claims **characterised in that**
a blocking device (20) in a condition of blocking engagement prevents the resetting movement of the drive unit (4, 6; 4, 5, 6),
the resetting spring (16) is stressed between a mounting body (17) and a rotary member (13),
wherein the rotary member (13) is rotated upon displacement of the drive unit (4, 6; 4, 5, 6),
and wherein the mounting body (17) is mounted rotatably relative to the casing (1, 10) and is in engagement with the drive unit (4, 6; 4, 5, 6) for rotary drive,
and that a rotational securing means which prevents rotary movement of the mounting body (17) relative to the casing (1, 10) is releasable.

7. A device according to one of the preceding claims **characterised in that** a blocking member (21) coupled to the drive unit (4, 6; 4, 5, 6) and a co-operating blocking member (24) coupled to the casing (1, 10) in a condition of blocking engagement prevent the resetting movement of the drive unit (4, 6; 4, 5, 6).

8. A device according to the preceding claim **characterised in that** to form the blocking engagement the blocking member (21) can be latched in latching positions with the co-operating blocking member (24) and there is provided a release element (28) to release the blocking engagement.

9. A device according to the preceding claim **characterised in that** the blocking member (21) forms an abutment (23) which after release of the blocking engagement comes to bear against the release member (28) and limits the resetting movement of the drive unit (4, 6; 4, 5, 6).

10. A device according to one of the three preceding claims **characterised in that** the blocking member (21) and the co-operating blocking member (24) are pressed against each other in the condition of blocking engagement by means of a spring (27).

11. A device according to one of the four preceding claims **characterised in that**
the dosing movement of the dosing member (13) is a rotary movement,
connected to the dosing member (13) is a shaft (15; 12) which also rotates upon a rotary movement of the rotary member (13),
the blocking member (21) is carried on the shaft (15; 12) in a condition of being prevented from rotating thereon, and
the co-operating blocking member (24) can be brought out of the condition of blocking engagement along the shaft (15; 12) against the force of a spring (27).

12. A device according to one of the preceding claims **characterised in that**
the dosing member (13) is mounted rotatably about an axis of rotation relative to the casing (1, 10) and projecting from the dosing member (13) in the direction of the axis of rotation is a shaft (15) at which the resetting spring (16) is supported.

13. A device according to one of the preceding claims **characterised in that**
a dosing knob (11) is supported rotatably by the casing (1, 10),
the dosing knob (11) is in engagement with the shaft (12) in such a way that a rotary movement of the dosing knob (11) causes a rotary movement of the shaft (12) and a longitudinal displacement of the shaft (12) relative to the dosing knob (11) is possible, and
the shaft (12) is in engagement with the dosing member (13), by virtue of which the dosing movement of the dosing member (13) is produced.

14. A device according to one of the preceding claims **characterised in that** the dosing member (13) and the resetting spring (16) are arranged beside the drive unit (4, 6; 4, 5, 6) in the casing (1, 10).

## Revendications

1. Dispositif d'administration dosée d'un produit injectable, ledit dispositif comprenant :
a) un boîtier (1, 10) avec un logement (2) pour un récipient qui contient le produit et qui reçoit un piston pouvant être poussé en direction d'une sortie (3) afin de distribuer une dose de produit choisie,
b) un élément doseur (13) permettant de réaliser un mouvement de dosage par rapport au boîtier (1, 10) afin de choisir la dose de produit,
c) une unité d'entraînement (4, 6 ; 4, 5, 6) qui est accouplée au boîtier (1, 10) et à l'élément doseur (13) de telle façon que l'unité d'entraînement (4, 6 ; 4, 5, 6) peut être déplacée par le mouvement de dosage de l'élément doseur (13) par rapport au boîtier (1, 10) d'une position de dosage initiale vers une position de dosage finale,
d) et un dispositif d'actionnement (10) qui fait effectuer à l'unité d'entraînement (4, 6 ; 4, 5, 6) un mouvement de distribution par lequel le piston est poussé en direction de la sortie (3),
**caractérisé en ce que**
e) un ressort de rappel (16) qui est bloqué dans un état tendu est accouplé par déblocage avec l'unité d'entraînement (4, 6 ; 4, 5, 6) et assure un mouvement de rappel de l'unité d'entraînement (4, 6 ; 4, 5, 6) en direction de sa position de dosage initiale.

2. Dispositif selon la revendication 1, **caractérisé en ce que** le ressort de rappel (16) est tendu par un déplacement de l'unité d'entraînement (4, 6 ; 4, 5, 6) effectué en direction de la position de dosage finale et un dispositif de verrouillage (20) empêche le mouvement de rappel de l'unité d'entraînement (4, 6 ; 4, 5, 6).

3. Dispositif selon l'une des revendications précédentes, **caractérisé en ce qu**'un ressort en spirale tendu entre le boîtier (1, 10) et un élément rotatif (13) pouvant tourner par rapport au boîtier (1, 10) forme le ressort de rappel (16), l'élément rotatif (13) tournant lors du déplacement de l'unité d'entraînement (4, 6 ; 4, 5, 6) et tendant ainsi le ressort de rappel (16).

4. Dispositif selon l'une des revendications précédentes, **caractérisé en ce que,** lors du mouvement de dosage, l'élément doseur (13) fait avancer par l'intermédiaire d'une commande à broche un élément mené (4) de l'unité d'entraînement (4, 6 ; 4, 5, 6) en direction du piston.

5. Dispositif selon la revendication précédente, **caractérisé en ce que** la commande à broche comprend un élément d'appui (6) qui est supporté en rotation par rapport au boîtier (1, 10) mais verrouillé de manière déblocable contre une torsion.

6. Dispositif selon l'une des revendications précédentes, **caractérisé en ce que** un dispositif de verrouillage (20) empêche le mouvement de rappel de l'unité d'entraînement (4, 6 ; 4, 5, 6) dans un engrènement de verrouillage,
le ressort de rappel (16) est tendu entre un palier (17) et un élément rotati f (13), l'élément rotatif (13) tournant lors du déplacement de l'unité d'entraînement (4, 6 ; 4, 5, 6),
et le palier (17) étant supporté en rotation par rapport au boîtier (1, 10) et étant en prise avec l'unité d'entraînement (4, 6 ; 4, 5, 6) pour un entraînement en rotation,
et **en ce que** une sécurité de torsion empêchant un mouvement de rotation du palier (17) par rapport au boîtier (1, 10) est déblocable.

7. Dispositif selon l'une des revendications précédentes, **caractérisé en ce qu**'un élément de verrouillage (21) couplé à l'unité d'entraînement (4, 6 ; 4, 5, 6) et un contre-élément de verrouillage (24) couplé au boîtier (1, 10) empêchent le mouvement de rappel de l'unité d'entraînement (4, 6 ; 4, 5, 6) dans un engrènement de verrouillage.

8. Dispositif selon la revendication précédente, **caractérisé en ce que** l'élément de verrouillage (21) est encliquetable dans des positions d'encliquetage avec le contre-élément de blocage (24) afin de réaliser l'engrènement de verrouillage et **en ce qu'**un élément de déblocage (28) est prévu pour débloquer l'engrènement de verrouillage.

9. Dispositif selon la revendication précédente, **caractérisé en ce que** l'élément de verrouillage (21) forme une butée (23) qui, après un déblocage de l'engrènement de verrouillage, vient en contact contre l'élément de déblocage (28) et limite le mouvement de rappel de l'unité d'entraînement (4, 6 ; 4, 5, 6).

10. Dispositif selon l'une des trois revendications précédentes, **caractérisé en ce que** l'élément de verrouillage (21) et le contre-élément de verrouillage (24) sont comprimés l'un contre l'autre au moyen d'un ressort (27) dans l'engrènement de verrouillage.

11. Dispositif selon l'une des quatre revendications précédentes, **caractérisé en ce que**
le mouvement de dosage de l'élément doseur (13) est un mouvement de rotation, un axe (15 ; 12) qui tourne également lors d'un mouvement de rotation de l'élément rotatif (13) est relié à l'élément doseur (13),
l'élément de verrouillage (21) est monté de manière sécurisée en torsion sur l'axe (15 ; 12), et
le contre-élément de verrouillage (24) peut être dégagé de l'engrènement de verrouillage le long de l'axe (15 ; 12) contre la force d'un ressort (27).

12. Dispositif selon l'une des revendications précédentes, **caractérisé en ce que** l'élément doseur (13) est supporté en rotation autour d'un axe de rotation par rapport au boîtier (1, 10) et **en ce qu'**un axe (15) sur lequel le ressort de rappel (16) s'appuie dépasse de l'élément doseur (13) dans le sens de l'axe de rotation.

13. Dispositif selon l'une des revendications précédentes, **caractérisé en ce que** un bouton de dosage (11) est supporté en rotation par le boîtier (1, 10), le bouton de dosage (11) est en prise avec un axe (12) de telle façon qu'un mouvement de rotation du bouton de dosage (11) provoque un mouvement de rotation de l'axe (12) et qu'un déplacement longitudinal de l'axe (12) par rapport au bouton de dosage (11) est possible,
et l'axe (12) est en prise avec l'élément doseur (13) de façon à générer le mouvement de dosage de l'élément doseur (13).

14. Dispositif selon l'une des revendications précédentes, **caractérisé en ce que** l'élément doseur (13) et le ressort de rappel (16) sont disposés dans le boîtier (1, 10) à côté de l'unité d'entraînement (4, 6 ; 4, 5, 6).
